# FASCICULE DE BREVET EUROPEEN

(11) **EP 0 392 124 B1**
(45) Date de publication et mention de la délivrance du brevet: **16.06.1993**
(21) Numéro de dépôt: 89401040.4
(22) Date de dépôt: 14.04.1989
(51) Int. Cl.: A61F 5/02

(54) **Implant chirurgical pour limiter le mouvement relatif des vertèbres**
Chirurgisches Implantat zum Begrenzen der Relativbewegung von Wirbeln
Surgical implant for limiting the relative movement of vertebrae

(43) Date de publication de la demande: 17.10.1990
(73) Titulaire: Bréard, Francis Henri, F-75014 Paris (FR)
(72) Inventeur: Bréard, Francis Henri, F-75014 Paris (FR)

(56) Documents cités:
- EP-A- 322 334
- DE-A- 3 113 142
- DE-A- 3 132 520
- FR-A- 1 004 625
- FR-A- 2 623 085
- FR-E- 59 969
- US-A- 3 648 691
- US-A- 4 554 914

## Description

La présente invention concerne un implant chirurgical destiné à prévenir un contact mutuel des vertèbres lors des flexions du rachis (colonne vertébrale).

On sait que, chez certains sujets dont les disques intervertébraux ont subi une usure importante, les nerfs rachidiens passant entre les vertèbres sont inévitablement écrasés sous l'effet du contact mutuel dur de ces dernières, survenant lors des flexions extrêmes vers l'avant ou vers l'arrière du rachis. Cet écrasement des nerfs rachidiens est à l'origine d'affections extrêmement douloureuses dont la plus fréquente est la sciatique.

Pour éliminer ce type d'affection, il est connu (voir FR-E-59969), de solidariser entre elles les apophyses des vertèbres à l'aide de plaquettes métalliques qui maintiennent en permanence un écartement suffisant entre vertèbres. Mais, bien entendu, en interdisant tout mouvement relatif des vertèbres, cette opération occasionne une gêne importante pour le patient, même si bien souvent elle ne concerne qu'un tronçon limité du rachis qui est en général celui qui couvre la région lombaire.

On connaît également une technique consistant à entrelacer un ligament artificiel autour des apophyses épineuses des vertèbres. Toutefois, cette technique, si elle permet aux vertèbres de conserver une certaine mobilité relative, a simplement pour effet d'interdire un contact des vertèbres à l'avant et est donc loin de résoudre le problème de l'écrasement des nerfs rachidiens qui se pose surtout à l'arrière.

L'état de la technique EP-A-322334, qui décrit une prothèse inter-épineuse composée dans une matière semi-élastique, comportant un oeillet de transfilage à son extrémité et des coussinets inter-épineux, est publié après la date de priorité de la présente demande.

C'est pourquoi la présente invention propose de résoudre ce problème, à l'aide d'un implant chirurgical tel que défini dans la revendication 1

Comme on le comprendra aisément, cette cale, une fois implantée, limite le mouvement de rapprochement des apophyses épineuses des vertèbres lors d'une flexion vers l'arrière du rachis, pour ainsi interdire tout contact mutuel des vertèbres au niveau de leur partie arrière, c'est-à-dire là où le problème de l'écrasement des nerfs rachidiens est prédominant.

A l'inverse, lors d'une flexion vers l'avant du rachis, les vertèbres peuvent librement s'écarter l'une de l'autre si bien que le patient ne ressentira pratiquement aucune gêne à la suite de l'implantation dans son dos d'une cale vertébrale selon l'invention.

Dans la pratique, les moyens de retenue de la cale seront conçus pour limiter le mouvement d'écartement des apophyses épineuses afin de prévenir un contact mutuel par l'avant des vertèbres, susceptible également de provoquer un écrasement de certains nerfs rachidiens, mais même dans ce cas la gêne occasionnée est tout à fait supportable pour le patient.

Dans sa forme la plus simple, la cale vertébrale conforme à l'invention est constituée part un petit plot insérable entre les apophyses épineuses de deux vertèbres successives. En cas de besoin, il sera bien entendu possible de réaliser l'implantation de plusieurs cales individuelles de ce type sur toute la longueur d'un tronçon du rachis comprenant plus de deux vertèbres, comme par exemple le tronçon lombaire.

La cale agit ainsi entre au moins deux vertèbres à la fois sans supprimer la possibilité d'écartement mutuel des apophyses épineuses de ces dernières.

Avantageusement, la cale présente, sur l'une et/ou l'autre de deux faces d'extrémité opposées, une gorge de réception et de guidage d'une apophyse respective, surdimensionnée par rapport à cette dernière, cette gorge présentant de préférence une largeur progressivement décroissante d'une extrémité à l'autre, pour se conformer à la forme naturelle effilée de l'apophyse qu'elle reçoit.

Selon une autre caractéristique avantageuse de l'invention, la cale comporte un ou plusieurs perçages transversaux à sa direction d'insertion, et de préférence deux perçages transversaux inclinés et croisés, ouverts à leurs deux extrémités.

Ce type de cale vertébrale est utilisée lorsque les moyens de retenue sont constitués par un ligament artificiel continu. Ce ligament, que l'on entrelace de façon lâche autour des apophyses épineuses des vertèbres en le faisant passer à travers les perçages de la ou des cales, assure, grâce à son élasticité propre, un arrêt en souplesse des vertèbres lors de la flexion vers l'avant du rachis.

Pour éviter que ce ligament soit entaillé lors de ces mouvements des vertèbres, il est de préférence prévu, selon l'invention, que chacun des perçages transversaux se termine à chaque extrémité par une ouverture évasée.

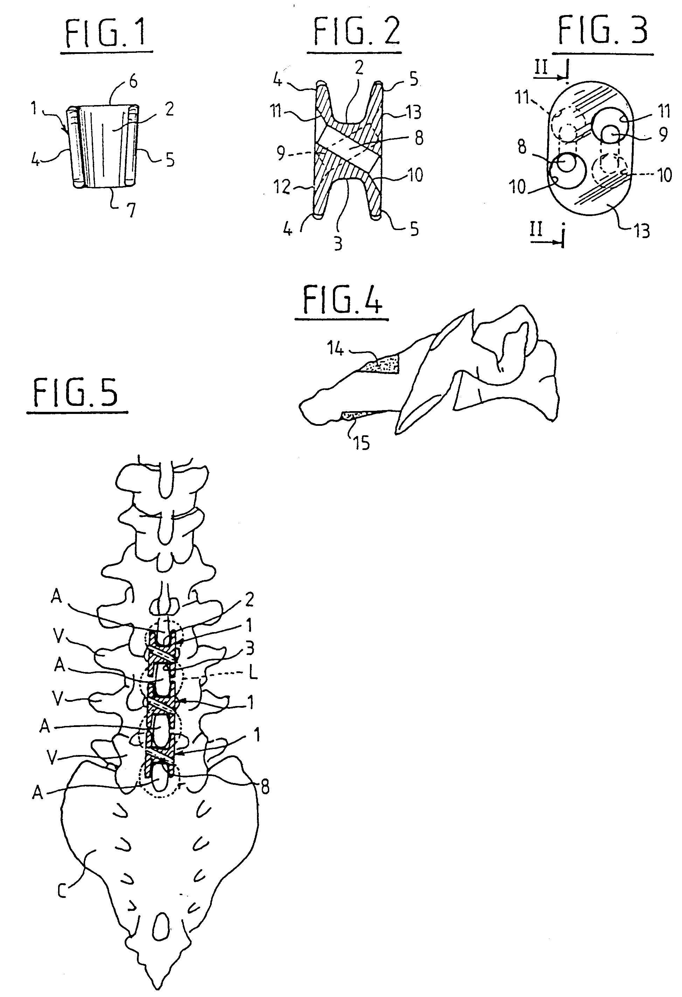

Plusieurs modes de réalisation de la présente invention vont maintenant être décrits plus en détails, mais uniquement à titre d'exemples non limitatifs, en référence aux dessins annexés dans lesquels :
. la figure 1 est une vue en plan d'une cale inter-vertébrale conforme au mode de réalisation de l'invention ;
. la figure 2 en est une vue en coupe transversale effectuée selon la ligne II-II de la figure 3 ;
. la figure 3 est une vue de côté de cette même cale inter-vertébrale ;
. la figure 4 est une représentation d'une vertèbre retouchée en vue de l'implantation d'une cale inter-vertébrale ;
. la figure 5 représente, en vue arrière, la partie basse d'un rachis sur lequel a été réalisée l'implantation de plusieurs cales inter-vertébrales conformes au mode de réalisation de l'invention ;

La cale inter-vertébrale 1, représentée sur les figures 1 à 3, présente la forme d'un petit plot muni, sur ses faces supérieure et inférieur, d'une gorge longitudinale respective 2 ou 3 délimitée par des lèvres de faible hauteur 4 et 5, qui, vues de côté, ont une forme en arc de cercle (figure 3). Comme le montre la figure 1, la largeur de chaque gorge 2 ou 3 diminue progressivement entre une face avant 6 et une face arrière 7 de la cale 1.

En outre, et comme le montrent les figures 2 et 3, la partie centrale de la cale inter-vertébrale 1 comporte deux perçages transversaux 8, 9 inclinés et croisés débouchant chacun à leurs extrémités, par des ouvertures 10, 11 s'évasant vers l'extérieur, sur les faces latérales 12, 13 de la cale 1. Comme le montre clairement la figure 3, les perçages 8, 9 sont simplement réalisés côte à côte sans aucune communication entre eux.

Une cale 1, comme décrite précédemment, est dimensionnée pour être insérée entre les apophyses épineuses de deux vertèbres successives. Chaque apophyse épineuse vient alors se loger, avec un léger jeu latéral, dans une gorge respective 2 ou 3 de la cale, après avoir été recalibrée comme représenté sur la figure 4 sur laquelle les fragments d'os découpés apparaissent en sombre sous les références 14 et 15.

Comme le montre la figure 5, plusieurs cales, telles que 1, peuvent être individuellement implantées entre les apophyses épineuses successives A des vertèbres V d'un tronçon donné du rachis, en l'occurrence le tronçon lombaire. Dans ce cas, les cales étagées 1 sont maintenues en place par un ligament artificiel continu L que l'on fait passer dans les perçages 8, 9 de chacune de ces dernières, où il se croise à chaque fois sur lui-même, et dont on entoure, de façon lâche, les apophyses des deux vertèbres lombaires extrêmes. Au-dessous de la vertèbre lombaire inférieure, le ligament L peut en variante être agrafé sur le coccyx C.

Une fois cette opération réalisée, les différentes cales 1 limitent le mouvement de rapprochement des apophyses lors d'une flexion vers l'arrière du rachis en interdisant dès lors tout contact dur entre les vertèbres et en supprimant donc le risques d'écrasement des nerfs rachidiens passant entre ces dernières, les lèvres 4, 5 des cales jouant, lors de ce mouvement de rapprochement, le rôle d'éléments de guidage latéraux. Lors d'une flexion du rachis en sens inverse, les apophyses des vertèbres pourront s'écarter l'une de l'autre, tout en restant à l'intérieur des gorges 2, 3 des cales, jusqu'à ce que les deux apophyses extrêmes entrent en contact souple avec le ligament L, qui limite ainsi le mouvement de rapprochement des têtes avant des vertèbres pour là encore prévenir un écrasement des nerfs rachidiens se trouvant à cet endroit.

Bien entendu, pour implanter les cales 1 ou 1a dans le rachis, il sera nécessaire au préalable de couper les ligaments naturels réunissant les vertèbres concernées par cette implantation.

Pour être complet, on précisera que chacune des cales décrites ci-dessus sera, de préférence, réalisée en une matière plastique à faible coefficient de frottement, tel que le polytétrafluoroéthylène, pour favoriser le glissement des apophyses des vertèbres à l'intérieur des cales.

Il va de soi aussi que, sur la base du principe de la présente invention, divers autres modes de réalisation de cette dernière peuvent être envisagés.

## Revendications

1. Implant chirurgical destiné à empêcher un contact mutuel des vertèbres lors des flexions du rachis, comprenant une cale inter-vertébrale (1, 1a) insérable entre les apophyses épineuses (A) d'au moins deux vertèbres successives (V) , ainsi qu'un ligament artificiel continu (L,), indépendant de la cale, pour la maintenir en place par rapport à ces apophyses, avec une possibilité de débattement en hauteur de ces dernières, ladite cale présentant, sur l'une et/ou l'autre de deux faces d'extrémité opposées, une gorge (2,3 ;) de réception et de guidage d'une apophyse respective (A), surdimensionnée par rapport à cette dernière.

2. Implant chirurgical selon la revendication 1, caractérisé en ce que chaque gorge (2, 3) présente une largeur progressivement décroissante d'une extrémité à l'autre.

3. Implant chirurgical selon la revendication 1 à 2, caractérisé en ce que les moyens de retenue comprennent un ligament (L) et la cale (1) comporte en outre, un ou plusieurs perçages (8, 9) transversaux à sa direction d'insertion et ouverts à leurs deux extrémités pour le passage de ce ligament (L).

4. Implant chirurgical selon la revendication 3, caractérisé en ce que la cale comporte deux perçages inclinés et croisés (8, 9).

5. Implant chirurgical selon la revendication 4, caractérisé en ce que chacun des perçages (8, 9) se termine, à chaque extrémité, par une ouverture évasée (10, 11).

## Patentansprüche

1. Chirurgisches Implantat zur Vermeidung einer gegenseitigen Berührung der Wirbel bei Beugung der Wirbelsäule, bestehend aus einem intervertebralen Keil (1; 1a), einfügbar zwischen die Apophysen (A) von mindestens zwei aufeinanderfolgenden Wirbeln (V), sowie einem vom Keil unabhängigen, ununterbrochenen, künstlichen Ligament (L), um den Keil zwischen diesen Apophysen festzuhalten, mit der Möglichkeit einer Bewegung der Apophysen nach oben, wobei der oben genannte Keil auf der einen bzw. der anderen von zwei gegenüberliegenden Seiten eine Führungs- und Aufnahmerille (2, 3) jeweils für eine Apophyse (A), mit größeren Abmessungen als diese, aufweist.

2. Chirurgisches Implantat entsprechend dem Patentanspruch 1, dadurch gekennzeichnet, daß die Breite jeder Rille (2, 3) vom einen Ende zum anderen abnimmt.

3. Chirurgisches Implantat entsprechend dem Patentanspruch 1 bis 2, dadurch gekennzeichnet, daß die Haltemittel aus einem Ligament (L) bestehen und der Keil (1) zudem quer zur Einführungsrichtung eine oder mehrere Bohrungen (8, 9) aufweist, wobei diese Haltemittel für den Einzug des Ligaments (L) an beiden Seiten offen sind.

4. Chirurgisches Implantat entsprechend dem Patentanspruch 3, dadurch gekennzeichnet, daß der Keil zwei über Kreuz liegende, schräge Bohrungen (8, 9) aufweist.

5. Chirurgisches Implantat entsprechend dem Patentanspruch 4, dadurch gekennzeichnet, daß die Bohrungen (8, 9) an beiden Enden (10, 11) jeweils konisch erweitert sind.

## Claims

1. A surgical implant intended to prevent physical contact between vertebrae during flexion of the spinal column, such implant comprising an intervertebral spacer (1, 1a) to be inserted between the processes (A) of at least two neighbouring vertebrae (V) in addition to a continuous artificial ligament (L), unattached to the spacer component and intended to keep it in position in relation to the processes, and allowing upward deflection of the latter, the spacer presenting on one and/or the other of its opposite faces a groove (2, 3) intended to receive and to guide the spines of the respective vertebrae (A) and of larger dimensions than the latter.

2. A surgical implant as described in Claim 1 and characterised by the fact that each groove (2, 3) presents a width which decreases progressively from one of its extremities toward the other.

3. A surgical implant as described in Claims 1 and 2, and characterised by the fact that the means of fixation comprising a ligament (L) and a spacer (1) include in addition one or more holes (8, 9) drilled along an axis transverse to the insertion direction of the implant and open at both ends in order to permit ligament (L) to pass through.

4. A surgical implant as described in Claim 3 and characterised by the fact that its spacer component includes two drilled holes whose axes are inclined at crossed angles (8, 9).

5. A surgical implant as described in Claim 4 and characterised by the fact that each of the drilled holes (8, 9) possesses at each end an opening of flared profile (10, 11).
